# EUROPEAN PATENT APPLICATION

(11) **EP 4 265 637 A1**
(43) Date of publication of application: **25.10.2023**
(21) Application number: 21905720.5
(22) Date of filing: 14.12.2021
(51) Int. Cl.: C07K 16/10, C12N 15/13, C12N 15/63, A61K 39/42, A61P 31/14, G01N 33/569, G01N 33/577

(54) **RESPIRATORY SYNCYTIAL VIRUS-SPECIFIC BINDING MOLECULE**

(30) Priority: 18.12.2020 CN 202011505001
(71) Applicant: Zhuhai Trinomab Pharmaceutical Co., Ltd., Zhuhai, Guangdong 519090 (CN)
(72) Inventor: ZHENG, Weihong, Zhuhai, Guangdong 519090 (CN); JIA, Zhenxing, Zhuhai, Guangdong 519090 (CN)
(74) Representative: Elzaburu S.L.P.
(86) International application number: PCT/CN2021/138018
(87) International publication number: WO 2022/127793

(57) **Abstract**

The present disclosure relates to a respiratory syncytial virus (RSV)-specific binding molecule and an application thereof. The present disclosure also provides a preparation method of the above molecule, and an application of the molecule in the preparation of a product that specifically binds to the RSV surface fusion glycoprotein and the preparation of an RSV vaccine, etc.

## Description

### Technical Field

The present disclosure relates to the fields of medicine and immunology, and specifically relates to a respiratory syncytial virus (RSV)-specific binding molecule, a preparation method of the above molecule, and use of the above molecule in the prevention and treatment of RSV infections.

### Background Art

Human respiratory syncytial virus (RSV) is widely distributed around the world and is one of the most common viral pathogens causing lower respiratory illness (LRI) in infants and young children, the elderly, and immunocompromised adults. Nearly all children will experience one or more infections by age 2, with the peak age of injection ranging from 2 to 8 months. RSV is the leading cause of lower respiratory tract infections and hospital visits during infancy and childhood. In hospitalized infants and young children, 40%-50% of bronchiolitis and 25% of pneumonia are caused by RSV infections. Furthermore, several studies show that severe infections in infants are a high-risk factor for subsequent asthma, which is far more severe than other microbial pathogens. Severe RSV infections also afflict the elderly. However, the continuous lack of specific treatment and safe and effective vaccines makes it difficult to reduce the morbidity and mortality of RSV infections globally.

Natural RSV infections produce insufficient immunity and cannot produce lasting immunity. Therefore, the distinguishing feature of RSV infections is that antibodies produced in the body by the previous infection cannot provide permanent protection. In the same epidemic season, different subtypes of RSV can cause re-infection. Even multiple natural RSV infections cannot induce the upper respiratory tract to produce lifelong immune protection against virus infection, so repeated infection is very common. Current methods for preventing and treating RSV infections include developed vaccines, antiviral compounds (ribavirin), antisense drugs, RNA interference, and antibody products such as immunoglobulins or monoclonal antibodies for intravenous injection. The monoclonal antibody palivizumab (Synagis^{®}) has been approved for the prevention and treatment of RSV in high-risk children. However, palivizumab is an expensive humanized monoclonal antibody that can only be used as a preventive treatment by passive immunization, and ribavirin, as a nucleoside antimetabolite, has serious toxicity and teratogenic effects.

Therefore, there is an urgent need to develop novel anti-RSV drugs, especially drugs that can treat RSV infections.

### Summary of the Invention

In order to solve the problems in the prior art, the present disclosure provides a separated human or humanized antibody or antigen-binding fragment thereof that specifically binds to the RSV surface fusion glycoprotein. In addition, the present disclosure also provides a nucleic acid sequence encoding the above antibody and antigen-binding fragment thereof; a vector including the above nucleic acid sequence and a corresponding host cell; and an application of the above neutralizing antibody in the prevention and treatment of RSV infections.

In a first aspect, the present disclosure provides a separated human or humanized antibody or antigen-binding fragment thereof that specifically binds to the RSV surface fusion glycoprotein, which includes a light chain variable region and a heavy chain variable region,
the heavy chain variable region includes: (1) complementarity determining region 1 (CDR1) having an amino acid sequence shown as SEQ ID NO: 1 or a functionally active CDR variant with an equivalent function, (2) CDR2 having an amino acid sequence shown as SEQ ID NO: 2 or a functionally active CDR variant with an equivalent function, and (3) CDR3 having an amino acid sequence shown as SEQ ID NO: 3 or a functionally active CDR variant with an equivalent function,
   and/or,
the light chain variable region includes: (1) CDR1 having an amino acid sequence shown as SEQ ID NO: 4 or a functionally active CDR variant with an equivalent function, (2) CDR2 having an amino acid sequence shown as SEQ ID NO: 5 or a functionally active CDR variant with an equivalent function, and (3) CDR3 having an amino acid sequence shown as SEQ ID NO: 6 or a functionally active CDR variant with an equivalent function. In a specific embodiment, the anti-RSV antibody or antigen-binding fragment thereof includes one, two or three CDRs (preferably three CDRs) of a VH region sequence of an antibody shown in Table 1. In some other embodiments, the antibody of the present disclosure includes one, two or three CDRs (preferably three CDRs) of a VL region sequence of the antibody shown in Table 1. In some embodiments, the antibody of the present disclosure includes 6 CDR sequences of the antibody shown in Table 1. In a preferred embodiment, the CDR sequences of the antibody are CDR sequences shown in Table 2. In some embodiments, the anti-RSV antibody or antigen-binding fragment thereof of the present disclosure includes a heavy chain variable region and a light chain variable region, the heavy chain variable region includes: (i) CDR1, including an amino acid sequence shown as SEQ ID NO: 1, or a sequence having amino acid substitution (e.g., conservative substitution), deletion or insertion of one or more and no more than 5 amino acids relative to the sequence, (ii) CDR2, including an amino acid sequence shown as SEQ ID NO: 2, or a sequence having amino acid substitution (e.g., conservative substitution), deletion or insertion of one or more and no more than 5 amino acids relative to the sequence, and (iii) CDR3, including an amino acid sequence shown as SEQ ID NO: 3, or a sequence having amino acid substitution (e.g., conservative substitution), deletion or insertion of one or more and no more than 5 amino acids relative to the sequence, the light chain variable region includes: (i) CDR1, including an amino acid sequence shown as SEQ ID NO: 4, or a sequence having amino acid substitution (e.g., conservative substitution), deletion or insertion of one or more and no more than 5 amino acids relative to the sequence, (ii) CDR2, including an amino acid sequence shown as SEQ ID NO: 5, or a sequence having amino acid substitution (e.g., conservative substitution), deletion or insertion of one or more and no more than 2 amino acids relative to the sequence, and (iii) CDR3, including an amino acid sequence shown as SEQ ID NO: 6, or a sequence having amino acid substitution (e.g., conservative substitution), deletion or insertion of one or more and no more than 5 amino acids relative to the sequence, and an anti-RSV antibody including a modified CDR still has the RSV-binding activity.

In some embodiments, the anti-RSV antibody or antigen-binding fragment thereof of the present disclosure includes a heavy chain variable region and a light chain variable region, the heavy chain variable region includes: (i) CDR1, consisting of an amino acid sequence shown as SEQ ID NO: 1, (ii) CDR2, consisting of an amino acid sequence shown as SEQ ID NO: 2, and (iii) CDR3, consisting of an amino acid sequence shown as SEQ ID NO: 3, and the light chain variable region includes: (i) CDR1, consisting of an amino acid sequence shown as SEQ ID NO: 4, (ii) CDR2, consisting of an amino acid sequence shown as SEQ ID NO: 5, and (iii) CDR3, consisting of an amino acid sequence shown as SEQ ID NO: 6.

In some embodiments, the anti-RSV antibody or antigen-binding fragment thereof of the present disclosure includes a heavy chain variable region VH and/or a light chain variable region VL,
the heavy chain variable region:
   1) includes an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or higher identity to an amino acid sequence shown as SEQ ID NO: 7, or consists of the above sequence, and an anti-RSV antibody including the above VH has the RSV-binding activity; or
   2) includes 3 heavy chain variable region CDRs of an antibody shown in Table 2, and has at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence shown as SEQ ID NO: 7, and an anti-RSV antibody including the above VH has the RSV-binding activity; or
   3) includes an amino acid sequence having one or more substitutions (e.g., conservative substitutions), insertions or deletions compared to the amino acid sequence shown as SEQ ID NO: 7, and an anti-RSV antibody including the above VH has the RSV-binding activity;
the above light chain variable region:
   1) includes an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or higher identity to an amino acid sequence shown as SEQ ID NO: 8, or consists of the above sequence, and an anti-RSV antibody including the above VL has the RSV activity; or
   2) includes 3 light chain variable region CDRs of the antibody shown in Table 2, and has at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence shown as SEQ ID NO: 8, and an anti-RSV antibody including the above VL has the RSV-binding activity; or
   3) includes an amino acid sequence having one or more substitutions (e.g., conservative substitutions), insertions or deletions compared to the amino acid sequence shown as SEQ ID NO: 8, and an anti-RSV antibody including the above VL has the RSV-binding activity. In preferred embodiments, the anti-RSV antibody of the present disclosure is an anti-RSV neutralizing antibody or antigen-binding fragment thereof.

In a preferred embodiment, the present disclosure provides an anti-RSV neutralizing antibody or antigen-binding fragment thereof, which includes a heavy chain variable region VH and a light chain variable region VL, the heavy chain variable region VH includes an amino acid sequence shown as SEQ ID NO: 7 or consists of the sequence, and the light chain variable region VL includes an amino acid sequence shown as SEQ ID NO: 8 or consists of the sequence.

In some embodiments, the present disclosure also covers an antigen-binding fragment of an anti-F protein neutralizing antibody, which includes, but is not limited to, Fv, Fab, Fab', Fab'-SH, F(ab')2, a double antibody, a linear antibody, a single-stranded antibody molecule (e.g., scFv), and a multispecific antibody consisting of antibody fragments.

In some embodiments, the above anti-RSV neutralizing antibody or antigen-binding fragment thereof also includes a heavy chain and/or light chain constant region sequence derived from a human antibody germline consensus sequence. The above light chain constant region is preferably a human κ or λ chain constant region. The heavy chain constant region may be a γ, µ, α, δ or ε chain constant region. In some embodiments, the above heavy chain constant region is of the human IgG1, IgG2, IgG3, IgG4, IgA1, IgA2, IgM, IgD or IgE isotype. Each heavy chain or light chain type is characterized by a specific constant region having a sequence well known in the art.

In some embodiments, the constant region is preferably a human IgG constant region such as a constant region of the human IgG1, IgG2, IgG3 or IgG4 isotype.

Preferably, the light chain of the monoclonal antibody of the present disclosure may be of the κ or λ type.

In a preferred embodiment, the light chain is of the λ type. The light chain may be a naturally occurring chain, including naturally rearranged, genetically modified, and synthesized light chains.

The heavy chain of the monoclonal antibody of the present disclosure may be selected from: IgM, IgA, and IgG isotypes, preferably IgG. In a preferred embodiment, the heavy chain of the monoclonal antibody is of the IgG type.

It is to be understood that sequence variants of these constant-region domains may also be used. For example, the sequence variants include one or more amino acid modifications at amino acid sites identified in accordance with the EU index system of Kabat et al. (1991). The above sites may be selected from 234, 235, 236, 237, 238, 239, 240, 241, 243, 244, 245, 246, 247, 248, 249, 250, 251, 252, 253, 254, 255, 256, 257, 258, 279, 280, 281, 282D, 283, 284, 285, 286, 287, 288S, 305, 306, 307, 308, 309, 310, 311, 312, 313, 315, 317, 339, 340, 341, 374, 376, 378, 380, 382, 383, 384, 385, 386, 387, 389, 426, 427, 428, 429, 430, 431, 432, 433, 434, 435, 436, 437, 438, 440, and 443. Alternatively, the Fc region includes non-naturally occurring amino acid residues known to those skilled in the art and located at other and/or alternative sites (examples include U.S. Patent No. 5,624,821; 6,277,375; 6,586,207; 6,737,056; 7,083,784; 7,317,091; 7,217,797; 7,276,585; 7,355,008; 2002/0147311; 2004/0002587; 2005/0215768; 2007/0135620; 2007/0224188; 2008/0089892; WO94/29351; WO99/58572; WO98/48032; WO03/073238; WO05/35727A2; WO05/74524A2; J. W. Chin et al., (2002), Journal of the American Chemical Society 124: 9026-9027; J. W. Chin and P. G. Schultz, (2002), ChemBioChem 11: 1135-1137; J. W. Chin, et al., (2002), PICAS United States of America 99: 11020-11024; and L. Wang and P. G. Schultz, (2002), Chem. 1-10).

In some embodiments, in order to prevent glycosylation of the above antibody, for example, a modification is made in the human IgG constant region, and the modification may be N297A or N297Q (Sazinsky, PNAS (2008), 105 (51): 20167-20172).

In some embodiments, in order to alter Fc receptor interactions, for example, a modification is made in the human IgG constant region, and the modification may be L234A and/or L235E or L235A.

In some embodiments, in order to prevent or reduce chain exchange, for example, a modification is made in the human IgG constant region, and the modification may be S228P (Angal, S., Mol Immunol (1993), 30: 105-108).

In some embodiments, in order to alter antibody-dependent cellular cytotoxicity (ADCC), and/or complement-dependent cytotoxicity (CDC), for example, a modification is made in the human IgG constant region, and the modification is known (including but not limited to: Natsume et al., Cancer Res (2008), 68 (10): 3863-72; Idusogie et al., J. Immunol (2001), 166 (4): 2571-5; Moore et al., mAbs (2010), 2 (2): 181-189; Lazar et al., PNAS (2006), 103 (11): 4005-4010; Shields et al., J. Biol. Chem. (2001), 276 (9): 6591-6604; Stavenhagen. Cancer Res (2007), 67(18): 8882-8890; Alegre et al., J. Immunol (1992) 148: 3461-3468.; and Kaneko, Niwa et al., Biodrugs (2011), 25 (1): 1-11).

In some embodiments, heterodimerization can also be induced by T366W modification, and optionally further by introduction of disulfide bonds through S354C and Y349C modifications on the opposing CH3 domain (Carter, Journal of Immunological Methods (2001), 248: 7-15).

In some embodiments, the above anti-RSV neutralizing antibody or antigen-binding fragment thereof specifically binds to the RSV surface fusion glycoprotein (F protein) that may be the pre-fusion conformational protein (Pre-F) of the RSV surface fusion glycoprotein (F protein). In a preferred embodiment, the neutralizing antibody binds to the RSV Pre-F protein with a KD of no more than 1×10⁻⁵ M, such as 1×10⁻⁶ M, 1×10⁻⁷ M, 1×10⁻⁸ M, 1×10⁻⁹ M, 1×10⁻¹⁰ M or less.

In particular, the RSV neutralizing antibody provided in the present disclosure is a humanized antibody or human antibody or single domain antibody.

In a second aspect, based on the above research findings, the present disclosure also provides a nucleic acid molecule encoding any of the above antibodies or fragments thereof. Based on the amino acid sequence of the above antibody molecule, those skilled in the art will readily obtain the nucleic acid molecule provided in the present disclosure according to common knowledge in the art. In an embodiment, the above nucleic acid molecule includes a nucleic acid molecule obtained due to codon degeneracy.

The nucleic acid molecule encoding the above antibody may be a naturally occurring nucleic acid that is derived from the germline or from rearrangements occurring in B cells, or the nucleic acid may be synthesized. The synthesized nucleic acid also includes a nucleic acid that has modified internucleoside bonds, including phosphorothioates, to increase the resistance of the nucleic acid to degradation. The nucleic acid can be genetically engineered or generated entirely synthetically by nucleotide synthesis.

In a preferred embodiment, the present disclosure provides a vector including at least one nucleic acid encoding the light chain of the monoclonal antibody of the present disclosure and/or at least one nucleic acid encoding the heavy chain of the monoclonal antibody of the present disclosure. The nucleic acids may be present in same vector or may be present in different vectors. Preferably, the vector includes a promoter operably linked to the nucleic acid to facilitate expression of the nucleic acid encoding the light chain and/or heavy chain. Preferably, the vector also includes an origin for replication and maintenance in host cells. The vector may also include a nucleotide sequence that encodes a signal sequence and is located at the 5' end of the nucleic acid encoding the light chain or heavy chain. The signal sequence may facilitate secretion of an encoded peptide chain into a medium.

Therefore, it is to be understood that a recombinant expression vector or expression cassette or transgenic cell line, recombinant bacteria, etc. that includes the above nucleic acid molecule shall also fall within the scope of protection of the present disclosure. Many prokaryotic and eukaryotic expression systems are known in the art, and eukaryotic host cells include, for example, yeast cells, insect cells, plant cells, and mammalian cells. Preferably, the mammalian cell is selected from a HEK293 cell, a PerC6 cell, a CHO cell, a COS cell, a HELA cell, and derivatives thereof. Particularly preferably, the mammalian cell is a human producer cell line.

In a preferred embodiment, the human monoclonal antibody of the present disclosure is derived from blood lymphocytes of a plasma sample with a high titer for the RSV F protein (RSV Pre-F and/or RSV Post-F), and a human antibody obtained in this way has a high affinity for RSV and has a neutralizing effect, thereby achieving effective protection against infections.

The present disclosure also provides a production method of a monoclonal antibody. In an embodiment, monoclonal antibodies are produced by culturing host cells containing the above expression vector. In an embodiment, produced monoclonal antibodies are secreted into a supernatant and may be purified by conventional chromatography.

In an embodiment, the present disclosure provides an anti-RSV antibody or antigen-binding fragment thereof prepared by the method of the present disclosure.

In a third aspect, the present disclosure also provides application of the above RSV-specific binding antibody in at least one of the following a) to d):
a) the preparation of a product that specifically binds to the RSV surface fusion glycoprotein;
b) the preparation of a product that specifically binds to RSV antigens;
c) the preparation of a product for the prevention, treatment or adjunctive treatment of RSV;
d) the preparation of an RSV vaccine.

Preferably, the above product is a drug.

In a preferred embodiment, the present disclosure further provides a drug for the prevention, treatment or adjunctive treatment of RSV infections, an active ingredient of which is the anti-RSV antibody or antigen-binding fragment thereof provided in the present disclosure.

Preferably, the antibody provided in the present disclosure can be prepared into a drug with a pharmaceutically acceptable carrier, and the drug can be administered by a variety of methods known in the art. A route and/or mode of administration may vary depending on the desired result.

In a fifth aspect, the present disclosure provides a composition including any of the anti-RSV antibodies or antigen-binding fragments thereof provided herein, preferably the composition is a pharmaceutical composition.

In an embodiment, the above composition also includes a pharmaceutically acceptable carrier. In an embodiment, the anti-RSV antibody or antigen-binding fragment thereof included in the composition is conjugated to a conjugation moiety. In an embodiment, the pharmaceutical composition further includes a pharmaceutically acceptable carrier, excipient or diluent.

In a sixth aspect, the present disclosure provides a method for preventing or treating a disease or symptoms associated with RSV infection, which includes the following steps: administering a prophylactically effective amount or therapeutically effective amount of the anti-RSV antibody of the present disclosure, or administering the pharmaceutical composition of the present disclosure to a subject.

The present disclosure also relates to a method for alleviating or improving symptoms associated with RSV infection, which includes the following steps: administering an effective amount of the anti-RSV antibody of the present disclosure, or administering the pharmaceutical composition of the present disclosure to a subject.

Administration can be performed by any suitable route, which includes, but is not limited to, topical, parenteral, local, and systemic administration, such as, intranasal, intramuscular administration, intradermal, intraperitoneal, intravenous, subcutaneous, and oral administration, or administration via the lung. In some embodiments, the pharmaceutical composition provided herein is administered by a nebulizer or inhaler. The anti-RSV antibody or pharmaceutical composition provided herein can be administered to any suitable subject such as a mammal such as a human.

In a seventh aspect, the present disclosure provides a method for detecting RSV in a subject or sample, which includes the following steps: (a) putting a subject or sample in contact with any of the anti-RSV antibodies or fragments thereof provided herein; and (b) detecting a complex composed of the anti-RSV antibody or fragment thereof and RSV. In a preferred embodiment, the anti-RSV antibody or antigen-binding fragment thereof of the present disclosure also includes a detectable label.

In an eighth aspect, the present disclosure relates to use of any of the anti-RSV antibodies or fragments thereof provided herein in the preparation of a drug or kit for preventing and treating a disease or symptoms associated with RSV infection in a subject.

In an embodiment, the present disclosure provides use of a composition including the above anti-RSV antibody in the preparation of a drug or kit for preventing and treating a disease or symptoms associated with RSV infection in a subject.

In a ninth aspect, the present disclosure provides a kit including the antibody or composition of the present disclosure, which is, for example, a diagnostic kit, detection kit or therapeutic kit.

The above preferred conditions can be combined with each other to obtain specific embodiments on the basis of common knowledge in the art.

### Brief Description of the Drawings

Fig. 1 shows results of flow sorting of specific memory B cells in PBMCs.
Fig.2 shows an SDS-PAGE map of non-reducing and reducing antibody proteins after purification.
Fig. 3 shows average expression levels of antibody TRN1021 and antibody TRN1022.
Fig. 4 shows results of the specific binding activity of antibody TRN1021 to purified RSV Pre-F and RSV Post-F.
Fig. 5 shows the in vitro micro-neutralizing activity of antibody TRN1021.
Fig. 6 shows the binding affinity of antibody TRN1021 for different concentrations of RSV Pre-F.
Fig. 7 shows results of a broad-spectrum neutralizing capacity assay of antibody TRN1021.
Fig. 8 shows results of virus titers in different tissues of cotton rats that are detected by different doses of antibody, wherein Fig. 8A and Fig. 8B are detection results of RSV A2 titers, and Fig. 8C and Fig. 8D are detection results of RSV 9320 titers.

### Detailed Description of the Invention

### I. Definitions

Before the present disclosure is described in detail below, it is to be understood that the present disclosure is not limited to the particular methods, schemes, and reagents described herein, as these may vary. It is also to be understood that the terms used herein are for the purpose of describing specific embodiments only and are not intended to limit the scope of the present disclosure, which shall be limited only by the appended claims. Unless otherwise defined, all technical and scientific terms used herein have the same meaning as commonly understood by those of ordinary skill in the art to which the present disclosure belongs.

For the purpose of interpreting the description, the following definitions will be used, and whenever appropriate, terms used in the singular may also include the plural, and vice versa. It is also to be understood that the terms used herein are for the purpose of describing specific embodiments only and are not intended to be limiting.

As used herein and unless otherwise specified, the term "about" or "approximate" means within plus or minus 10% of a given value or range. Where an integer is required, the term means rounding up or down to the nearest integer within plus or minus 10% of a given value or range.

When used in combination with a numerical value, the term "about" is intended to cover numerical values within a range having a lower limit that is 5% less than the specified numerical value and an upper limit that is 5% greater than the specified numerical value. The term "and/or" is to be understood to mean either or both of the alternatives.

As used herein, the term "comprise" or "include" is intended to include the elements, integers or steps described herein, but does not exclude any other elements, integers or steps. Herein, when used, unless otherwise specified, the terms "comprise" or "include" also covers the situation consisting of the described elements, integers or steps.

For example, when referring to an antibody variable region "including" a certain specific sequence, it is also intended to cover an antibody variable region consisting of that specific sequence.

Provided herein are an antibody (e.g., monoclonal antibody) and an antigen-binding fragment thereof that specifically bind to RSV. Specifically, provided herein is an anti-RSV monoclonal antibody that specifically binds to RSV and the anti-RSV antibody includes variants of the parent antibody. Specifically, provided herein is an antibody that specifically binds to RSV (e.g., human RSV). Particularly, provided herein is an anti-RSV antibody that includes modifications in one or more amino acid residues (e.g., one or more amino acid substitutions in the framework region of the heavy chain variable region) and retains affinity for an antigen compared to the parent antibody without the above modifications.

The term "respiratory syncytial virus" or "RSV" is a single-stranded negative-strand RNA virus, belonging to the family *Paramyxoviridae* and the genus *Pneumoviridae.* Because the virus can cause unique cell fusion in cultured cells, it is named respiratory syncytial virus (RSV). RSV can be divided into RSV-A and RSV-B subtypes according to the differences in surface antigens. The virus is spread by airborne droplets and close contact. RSV contains 10 genes encoding 11 proteins, of which the surface fusion glycoprotein (F protein) and attachment glycoprotein (G protein) are the most important virus antigens that stimulate the body to produce protective antibodies. The G protein differs greatly between the subtypes, and the F protein is highly conserved between the subtypes, directly mediates the fusion of the virus and cells, the penetration of the virus, and the formation of syncytia, and thus is the main target protein that stimulates the body to produce protective antibodies. Herein, "respiratory syncytial virus" or "RSV" refers to any respiratory syncytial virus or RSV molecule known to those skilled in the art. For example, RSV may include any of the above subtypes. For example, RSV may be derived from a mammal. For example, RSV may be derived from a human.

The F protein is an N-glycosylated type I transmembrane protein of 574 amino acids in full length, having a cleaved signal peptide at the N-terminus and a membrane anchor near the C-terminus. The amino acid sequence of the F protein is provided in GenBank under accession number AAX23994. The F protein has a pre-fusion conformation (Pre-F) and a post-fusion conformation (Post-F) on the surface of the virion. Pre-F exists as a trimer and undergoes a conformational change that results in the insertion of a hydrophobic fusion peptide into the host cell membrane and subsequent refolding of the F protein into a stable and elongated post-fusion conformation (Post-F), leading to the fusion of the virus and the host cell membrane.

The term "laboratory strain" refers to a strain of RSV that has been passaged extensively in in-vitro cell culture. The "laboratory strain" may obtain adaptive mutations that may affect their biological properties. The term "clinical strain", "clinical isolate" or "clinical sample isolate" refers to an RSV isolate, such as RSV subtype A or subtype B, obtained from an infected individual, which has been separated and grown in tissue culture by a limited number of passages.

The term "antibody" is used herein in the broadest sense and covers a variety of antibody structures, including, but not limited to, monoclonal antibodies, polyclonal antibodies, multispecific antibodies (e.g., bispecific antibodies), and antibody fragments, so long as they exhibit the desired antigen-binding activity. An intact antibody typically includes at least two full-length heavy chains and two full-length light chains, and may include fewer chains in some cases. For example, an antibody naturally occurring in a camel may include a heavy chain only. Antibodies may be humanized or human antibodies and single domain antibodies such as VH, VHH or VL. Examples of antibody fragments include, but are not limited to, Fab, Fab', F(ab')2, single-chain Fv (scFv), Fv, dsFv, double antibody, Fd and Fd' fragments, and other fragments, including modified fragments (e.g., Methods in Molecular Biology, Vol 207: Recombinant Antibodies for Cancer Therapy Methods and Protocols (2003); Chapter 1; p 3-25, Kipriyanov). The above fragments may include multiple chains linked together, for example, by disulfide bonds and/or peptide adaptors. An antibody fragment generally includes at least or about 50 amino acids, and typically at least or about 200 amino acids.

As used herein, the term "monoclonal antibody" or "mAb" refers to an antibody derived from, for example, a single copy or clone of a eukaryotic, prokaryotic, or phage clone, that is, antibodies composing the above population are identical and/or bind to the same epitope except for possible variant antibodies (e.g., variant antibodies including natural mutations or produced during production of a monoclonal antibody product) which are usually present in very small amounts. The modifier "monoclonal" refers to the characteristic that an antibody is obtained from a substantially homogeneous population of antibodies, and is not to be construed as requiring the production of the antibody by any particular method. Monoclonal antibodies can be produced, for example, by a hybridoma technique, a recombinant technique, a phage display technique, a synthesis technique such as CDR grafting, or a combination of these techniques or other techniques known in the art.

The term "natural antibody" refers to naturally occurring immunoglobulin molecules having different structures. A "natural sequence Fc domain" includes an amino acid sequence that is identical to the amino acid sequence of an Fc domain found in nature. Natural sequence human Fc domains include, for example, natural sequence human IgG1 Fc domains (non-A and A allotypes); a natural sequence human IgG2 Fc domain; a natural sequence human IgG3 Fc domain; a natural sequence human IgG4 Fc domain; and naturally occurring variants thereof.

The term "human antibody" refers to an antibody having an amino acid sequence corresponding to the following antibodies, and the above antibody is produced by a human or human cell or derived from a non-human source, which utilizes a human antibody library or other human antibody encoding sequences. The term "human antibody" explicitly excludes humanized antibodies including non-human antigen-binding residues. The term "neutralizing antibody" refers to an antibody that reduces or inhibits at least one biological activity of the F protein. For example, the neutralizing antibody blocks the fusion of RSV and host cells, prevents the formation of syncytia, and prevents primary disease caused by RSV. Alternatively, the neutralizing antibody of the present disclosure can improve at least one symptom of RSV infection. The reduction in biological activity may be a partial reduction or a complete reduction. The degree to which an antibody neutralizes RSV is referred to as the neutralizing potency of the antibody. The neutralizing efficacy of an antibody can be determined or measured using one or more assays known to those of ordinary skill and/or referred to herein, including, but not limited to, the competitive binding assay, the direct and indirect sandwich assays, the immunoprecipitation assay, the enzyme-linked immunosorbent assay (ELISA), the plaque reduction test, the micro-neutralization test, the immobilized virus diluted antiserum test, and the pseudovirus neutralization test.

The term "complementarity determining region" or "CDR" or "hypervariable region" is an amino acid region in an antibody variable region that is primarily responsible for binding to an antigenic epitope. CDRs of a heavy chain and a light chain are commonly referred to as CDR1, CDR2, and CDR3, numbered sequentially from the N-terminus.

Various schemes are known in the art for determining CDR sequences of a given VH or VL amino acid sequence: Kabat complementarity determining regions (CDRs) are determined based on sequence variability and are most commonly used (Kabat et al., Sequences of Proteins of Immunological Interest, 5th Edition, Public Health Service, National Institutes of Health, Bethesda, Md. (1991)), while Chothia refers to the position of the structural loop (Chothia et al., (1987) J. Mol. Biol. 196: 901-917; and Chothia et al., (1989) Nature 342: 877-883), AbM CDRs are a compromise between Kabat CDRs and Chothia structural loops, and are used by Oxford Molecular's AbM antibody modeling software, and "Contact" CDRs are determined based on analysis of available complex crystal structures. Residues of each CDR are shown below according to different CDR determination schemes.

| CDR | Kabat scheme | Abm scheme | Chothia scheme | Contact scheme |
|---|---|---|---|---|
| CDR1 | L24-L34 | L24-L34 | L26-L32 | L30-L36 |
| CDR2 | L50-L56 | L50-L56 | L50-L52 | L46-L55 |
| CDR3 | L89-L97 | L89-L97 | L91-L96 | L89-L97 |
| CDR1 | H31-H35B | H26-H35B | H26-H32 | H31-H35B |
| (Kabat numbering system) | | | | |
| CDR1 | H31-H35 | H26-H32 | H26-H32 | H31-H35 |
| (Chothia numbering system) | | | | |
| CDR2 | H50-H65 | H50-H58 | H53-H55 | H47-H58 |
| CDR3 | H95-H102 | H95-H102 | H96-H101 | H93-H101 |
| (Kabat numbering system) | | | | |

In an embodiment, the CDRs of the antibody of the present disclosure are CDR sequences located at the following Kabat residue sites according to the Kabat numbering system: sites 26-33 (CDR1), sites 51-58 (CDR2), and sites 97-116 (CDR3) in VH, and sites 27-32 (CDR1), sites 50-52 (CDR2), and sites 89-98 (CDR3) in VL.

A CDR can also be determined based on sites having the same Kabat number as a reference CDR sequence (e.g., any of the exemplary CDRs of the present disclosure).

The term "functionally active CDR variant" refers to an amino acid sequence that retains the function (i.e., the ability to bind to a corresponding fragment of RSV) of the parent CDR and differs in sequence from the parent CDR sequence, which may have at least one amino acid residue modification relative to the parent CDR, or has at least 60%, 65%, 70%, 75%, 80%, 85%, 90%, 95%, 96%, 97%, 98%, 99% or higher sequence identity to the parent CDR sequence.

Amino acid residue modifications may be chemical changes or sequence alterations in an amino acid sequence, which retain the biological properties of the parent sequence. A sequence alteration may be deletion, substitution, insertion of one to several amino acid residues such as 1, 2, 3, 4 or 5 amino acid residues, or addition of an amino acid sequence, or chemical derivatization of one to several amino acid residues such as 1, 2, 3, 4 or 5 amino acid residues, or a combination thereof. Substitution of amino acid residues may be conservative substitution.

The term "conservative substitution" refers to the substitution of an amino acid with another amino acid within the same category. For example, an acidic amino acid is substituted with another acidic amino acid, a basic amino acid is substituted with another basic amino acid, or a neutral amino acid is substituted with another neutral amino acid. The term "variant" related to an antibody herein refers to an antibody that includes a target antibody region (e.g., a heavy chain variable region or light chain variable region or heavy chain CDR or light chain CDR) having an amino acid alteration due to substitution, deletion and/or insertion of at least one amino acid residue (e.g., 1-30, or 1-20, or 1-10, such as 1 or 2 or 3 or 4 or 5 amino acid residues), or chemical derivatization of one or more amino acid residues, and the variant substantially retains the biological properties of the unaltered antibody molecule. In an aspect, the present disclosure covers variants of any of the antibodies mentioned herein. In an embodiment, the antibody variant retains at least 60%, 70%, 80%, 90%, or 100% of the biological activity (e.g., antigen-binding capacity) of the unaltered antibody. It is to be understood that the heavy chain variable region or the light chain variable region, or each CDR region of an antibody may be altered individually or in combination. In some embodiments, in one or more or all three heavy-chain CDRs, no more than 1, 2, 3, 4, 5, 6, 7, 8, 9 or 10 amino acids are altered. Preferably, the above amino acid alteration is amino acid substitution, preferably conservative substitution. In some embodiments, the antibody variant has at least 60%, 65%, 70%, 75%, 80%, 85%, 90%, 95%, 96%, 97%, 98%, 99% or higher sequence identity to the parent antibody in a target antibody sequence region.

The term "separated" antibody refers to an antibody that has been separated from its components of natural environment. In some embodiments, the antibody is purified to more than 95% or 99% purity as determined by, for example, electrophoresis (e.g., SDS-PAGE, isoelectric focusing (IEF), and capillary electrophoresis) or chromatography (e.g., ion exchange and reverse phase HPLC). For a review of methods for assessing antibody purity, see, for example, Flatman et al., J. Chromatogr. B848: 79-87 (2007).

The term "separated" nucleic acid refers to a nucleic acid molecule that has been separated from its components of natural environment. A separated nucleic acid includes a nucleic acid molecule that is contained in a cell that normally contains the nucleic acid molecule, but the nucleic acid molecule is present outside the chromosome or at a chromosomal locus different from its natural chromosomal locus.

The term "affinity" or "binding affinity" refers to the intrinsic binding affinity that reflects the interaction between members (e.g., an antibody and an antigen) of a binding pair. The affinity of a molecule X for its partner Y can generally be expressed in terms of an equilibrium dissociation constant (KD). The equilibrium dissociation constant is a ratio of a dissociation rate constant to a binding rate constant (kdis and kon, respectively). A smaller KD indicates a smaller dissociation, representing a stronger affinity of an antibody for an antigen. Affinity can be measured by conventional methods known in the art. For example, KD is determined in a BIACORE instrument by surface plasmon resonance (SPR). Typically, an antibody (e.g., the neutralizing antibody TRN1021 of the present disclosure) dissociates from an antigen (e.g., the F protein of RSV) with an equilibrium dissociation constant (KD) of no more than 1×10⁻⁵ M, such as 1×10⁻⁶ M, 1×10⁻⁷ M, 1×10⁻⁸ M, 1×10⁻⁹ M, 1×10⁻¹⁰ M or less.

The term "immunoconjugate" refers to an antibody conjugated to one or more heterologous molecules, including but not limited to carriers. The term "pharmaceutical composition" refers to a preparation that is in a form permitting the biological activity of an active ingredient contained therein to be effective and does not contain other ingredients that have unacceptable toxicity to a subject to whom the preparation is administered.

The term "pharmaceutically acceptable carrier" refers to one or more non-toxic materials that do not interfere with the biological activity of an active ingredient when administered with a therapeutic agent, including, but not limited to, buffers, preservatives, compatible carriers, diluents, adjuvants (e.g., Freund's adjuvants (complete and incomplete)), excipients, vehicles, and optionally other additives or encapsulating substances. Pharmaceutically acceptable carriers applicable to the present disclosure may be conventional pharmaceutical preparation excipients, and compositions and preparations suitable for delivery of the disclosed neutralizing antibody.

The terms "treatment" refers to slowing, improving, alleviating, stopping, reducing the progression, severity, and/or duration of an upper and/or lower respiratory tract RSV infection, or symptoms or respiratory conditions (e.g., asthma, wheezing, and a combination thereof) associated therewith. In certain embodiments, the term refers to reduction or inhibition of replication of RSV inhibition or reduction of spread of RSV to other tissues or subjects (e.g., spread to the lower respiratory tract), inhibition or reduction of infection of cells by RSV, or improvement of one or more symptoms associated with upper and/or lower respiratory tract RSV injections.

The term "prevention" refers to preventing or inhibiting the development or onset of upper and/or lower respiratory tract RSV injections or a respiratory condition associated therewith in a subject; preventing or inhibiting the progression of an upper respiratory tract RSV infection to a lower respiratory tract RSV infection due to an applied therapy (e.g., a prophylactic or therapeutic agent) or a respiratory condition associated therewith; preventing symptoms of upper and/or lower respiratory tract RSV infections or a respiratory condition associated therewith; or applying a combination of therapies (e.g. a combination of prophylactic or therapeutic agents).

The term "effective amount" refers to an amount or dose that is sufficient to achieve or at least partially achieve the desired effect after single or multiple administration. The term "therapeutically effective amount" refers to an amount that produces the desired effect in a treated subject, including improvement of conditions of the subject (e.g., improvement of one or more symptoms), and/or delay of the progression of symptoms, etc. The term "prophylactically effective amount" refers to an amount that is sufficient to prevent, stop or delay the onset of a disease. Determination of an effective amount is well within the ability of those skilled in the art. For example, a therapeutically effective amount depends on a specific disease involved; the extent or severity of the disease; response in an individual patient; a specific antibody to be administered; a mode of administration; the bioavailability characteristics of a preparation to be administered; a selected dosing regimen; the use of any concomitant therapy, etc.

The term "vaccine" or "vaccine composition" refers to a composition including at least one immunogenic composition that induces an immune response in an animal.

The term "subject" or "individual" is a primate (e.g., humans and non-human primates such as monkeys). In certain embodiments, the individual or subject is a human.

### II. Detailed description of specific embodiments

In an aspect, the present disclosure provides an antibody or antigen-binding fragment thereof that specifically binds to the RSV surface fusion glycoprotein, which includes:
(a) the following CDRs of a heavy chain variable region:
   CDR1, including a sequence shown as SEQ ID NO: 1;
   CDR2, including a sequence shown as SEQ ID NO: 2;
   CDR3, including a sequence shown as SEQ ID NO: 3,
(b) the following CDRs of a light chain variable region:
   CDR1, including a sequence shown as SEQ ID NO: 4;
   CDR2, including a sequence shown as SEQ ID NO: 5;
   CDR3, including a sequence shown as SEQ ID NO: 6.

In an aspect, the present disclosure provides a separated antibody or antigen-binding fragment thereof that specifically binds to the RSV surface fusion glycoprotein, which includes three CDRs of a heavy chain variable region shown as SEQ ID NO: 7 and three CDRs of a light chain variable region shown as SEQ ID NO: 8.

In the antibody or antigen-binding fragment thereof according to any one of the above aspects, the heavy chain variable region is selected from:
(1) a sequence that includes the heavy chain CDRs according to any one of the above aspects and has at least 80% identity to SEQ ID NO: 7; or
(2) a sequence shown as SEQ ID NO: 7.

In the antibody or antigen-binding fragment thereof according to any one of the above aspects, the light chain variable region is selected from:
(1) a sequence that includes the light chain CDRs according to any one of the above aspects and has at least 80% identity to SEQ ID NO: 8; or
(2) a sequence shown as SEQ ID NO: 8.

The antibody or antigen-binding fragment thereof according to any one of the above aspects includes a heavy chain variable region and a light chain variable region, wherein the heavy chain variable region includes a sequence shown as SEQ ID NO: 7, the light chain variable region includes a sequence shown as SEQ ID NO: 8.

The separated antibody according to any one of the above aspects is an IgG antibody.

The antibody or antigen-binding fragment thereof according to any one of the above aspects, wherein the antigen-binding fragment is selected from Fab, Fab'-SH, Fv, scFV, or (Fab')2 fragments.

The antibody or antigen-binding fragment thereof according to any one of the above aspects includes a constant region sequence, wherein at least a portion of the constant region sequence is a human consensus constant region sequence.

In an aspect, the present disclosure provides a nucleic acid molecule encoding the antibody or antigen-binding fragment thereof according to any one of the above aspects. In an aspect, the present disclosure provides a vector including the nucleic acid molecule according to the above aspect.

In some preferred embodiments, the vector is an expression vector.

In an aspect, the present disclosure provides a host cell including the above vector.

In some embodiments, the host cell is a prokaryote or eukaryote;

In some embodiments, the host cell is selected from an *Escherichia coli* cell, a yeast cell, a mammalian cell, and other cells applicable to the preparation of an antibody or antigen-binding fragment thereof, and the mammalian cell is, for example, a CHO cell, HEK293 cell or COS cell.

In an aspect, the present disclosure provides a production method of an antibody or antigen-binding fragment thereof that binds to the RSV surface fusion glycoprotein, which includes: the above host cell is cultured under conditions suitable for expression of a nucleic acid encoding the antibody or antigen-binding fragment thereof according to any one of the above aspects, optionally the antibody or antigen-binding fragment thereof is separated, and optionally produced antibodies or antigen-binding fragments thereof are collected.

The present disclosure provides an antibody or antigen-binding fragment thereof prepared by the method according to the above aspect.

In an aspect, the present disclosure provides a pharmaceutical composition including the above antibody or antigen-binding fragment thereof and a pharmaceutically acceptable carrier.

The pharmaceutical composition according to the above aspect also includes other therapeutic agents. It is to be understood that the neutralizing antibody or pharmaceutical composition provided in the present disclosure can be integrated into suitable delivery carriers, excipients, and other agents in a preparation for co-administration to provide improved transfer, delivery, tolerance, etc.

In an aspect, the present disclosure provides use of the antibody or antigen-binding fragment thereof according to any one of the above aspects in the preparation of a drug for preventing or treating a disease or symptoms associated with RSV injections.

The drug of the present disclosure must be sterilized and stable under the conditions of preparation and storage. In a case that sterile powder for sterile injectable solution is prepared, preferred preparation methods are vacuum drying and lyophilization, by which powder of an active ingredient and other desired ingredients is yielded from a pre-sterilized and filtered solution of the active ingredient and other desired ingredients. Alternatively, the drug of the present disclosure may be in a solution, and may be added to and/or mixed with a suitable pharmaceutically acceptable excipient before delivery or during delivery to provide an injectable unit dosage form. Preferably, the pharmaceutically acceptable excipients used in the present disclosure are suitable for high drug concentrations, may maintain proper fluidity, and may delay absorption if desired.

In an aspect, the present disclosure provides a method for preventing or treating an RSV infection-associated disease, which includes the following steps: an effective amount of the above antibody or antigen-binding fragment thereof is administered to a subject in need.

In an embodiment, the neutralizing antibody of the present disclosure can produce 100% inhibition of the virus.

In an aspect, the present disclosure provides a method for detecting the presence of RSV in a sample, which includes the following steps: a sample is put in contact with the above antibody or antigen-binding fragment thereof.

For purposes of clarity and conciseness, features are described herein as part of the same or separate embodiments. However, it is to be understood that the scope of the present disclosure may include some embodiments having combinations of all or some of the described features.

### Examples

### Example 1: screening, expression, and purification of an RSV neutralizing antibody

### 1. Expression and identification of RSV Pre-F and RSV Post-F proteins

Sequences of a pre-fusion conformation (Pre-F) and a post-fusion conformation (Post-F) of the F protein of a target gene RSV A2 strain were found in the NCBI database, eukaryotic expression vectors were constructed, and the proteins were expressed and purified. The proteins were detected by PAGE, and RSV Pre-F protein and RSV Post-F protein consistent with the naturally occurring trimeric RSV F protein were known and obtained.

Then, the proteins were tested by ELISA. Results show that both RSV Pre-F protein and RSV Post-F protein positively bind to mouse anti-RSV fusion protein antibodies and do not bind to other irrelevant antibodies. Therefore, the expressed RSV Pre-F protein and RSV Post-F protein of this example are both natural trimeric proteins with specific binding activity.

### 2. Screening of positive plasma samples and separation of memory B cells

Peripheral blood mononuclear cells (PBMCs) were separated from a peripheral venous blood sample collected from a healthy adult volunteer by Ficoll density gradient centrifugation. Plasma samples with high titers against RSV F protein (RSV Pre-F and RSV Post-F) were screened by ELISA. Plasma samples with high antibody titers were screened out, and PBMCs in the samples were subjected to flow sorting.

Sorting of memory B cells by flow cytometry: PBMCs thawed in a water bath at 37°C were filtered with a 40 µm filter, and single plasma cells were sorted by using a flow cytometry (manufacturer: BD, model: FACSria) according to gating set as follows: CD3-PE-Cy5-/CD16-PE-Cy5-/CD235a-PE-Cy5-/CD14-FITC-/IgD-PE-/CD20-APC+/CD 27-APC-H7+RSV Post-BV421+/RSV pre-PE-Cy7+, the population of memory B cells specifically against the RSV F protein (Pre-F and/or Post-F) were sorted from the PBMCs, morphologically intact single cells were selected and placed in a 96-well PCR plate (each well contained 20 µL of single cell lysate), each well contained one memory B cell, and the plate was stored in a refrigerator at -80°C for later use.

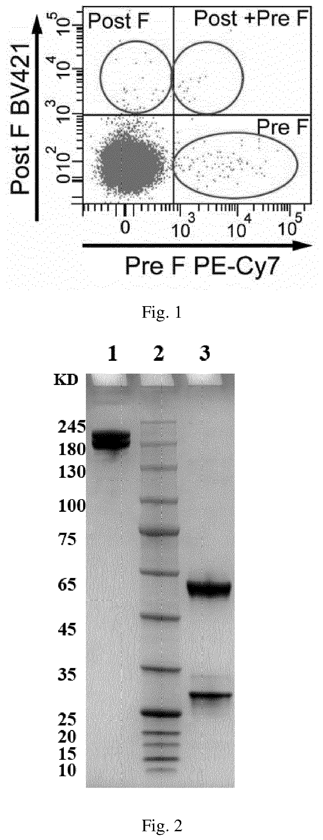

Sorting results are shown in Fig. 1. Memory B cells in a candidate sample were sorted by flow cytometry, and memory B cells specifically binding to Pre-F and/or Post-F proteins were screened out.

### 3. Separation of antibody variable region genes

(1) RT-PCR: 0.5 µM primers for heavy chain and light chain constant regions of different subtypes (the primers were designed at specific sites by a conventional method, see CN107760690 B) and Superscript III reverse transcriptase were added into the 96-well plate containing single B cells, the mixture was incubated at 37°C for 1 h, and PCR amplification was performed according to the following conditions: pre-denaturation was performed at 95°C for 15 min; then, denaturation was performed at 95°C for 1 min, annealing was performed at 55°C for 1 min, extension was performed at 72°C for 1 min, 30 cycles were performed; finally extension was performed 72°C for 10 min; and storage was performed at 4°C for 5 min; and an obtained product cDNA was stored at -20°C.
(2) PCR: 50 µL of reaction system contained 5 µL of reverse transcription product, HotStarTaq Plus enzyme, dNTPs, and 0.5 µM specific primers for heavy chain and light chain variable regions of different subtypes (the primers were designed at specific sites by a conventional method, see CN107760690B), and PCR amplification was performed according to the following conditions: pre-denaturation was performed at 94°C for 5 min; then, denaturation was performed at 94°C for 30 s, annealing was performed at 55°C for 30 s, and extension was performed at 72°C for 50 s, 35 cycles were performed; and finally extension was performed at 72°C for 7 min.

An obtained PCR product was detected by 1% agarose gel electrophoresis, and the remaining PCR product was purified by using a Qiagen PCR Purification Kit (Qiagen).

### 4. Construction of an expression vector for the recombinant antibody and expression of antibody

The PCR product of the antibody variable region genes that was identified as positive by gel electrophoresis and had paired heavy chain and light chain, was linked to a pcDNA3.3 vector by TA cloning to construct an expression vector for the fully human RSV neutralizing antibody, then the expression vector was transformed into DH5α competent bacteria, the bacteria were cultured on a plate containing ampicillin at 37°C overnight, 10 single colonies were picked and subjected to PCR by using specific primers under the following reaction conditions: pre-denaturation was performed at 94°C for 3 min; then, denaturation was performed at 94°C for 30 s, annealing was performed at 55°C for 30 s, extension was performed at 72°C for 100 s, 28 cycles were performed; and finally extension was performed at 72°C for 5 min. 5 uL of PCR product was detected by 1% agarose gel electrophoresis. Results show that transformants containing the antibody heavy chain and light chain genes were identified in positive transformants. HEK293 cells were co-transfected with the expression vector for the antibody heavy chain and light chain that positively bound to the antigen, the old medium was replaced with a large amount of fresh medium 6-8 h after transfection, the cells were cultured an incubator with 8% CO₂ at 37°C for expression of antibodies, and a fully human anti-RSV monoclonal antibody TRN1021 was obtained, with a sequence shown in the following Tables 1 and 2:

**Table 1 Heavy chain variable region sequence and light chain variable region sequence of antibody TRN1021**

| | | |
|---|---|---|
| VH | SEQ ID No. 7 | QVQLVQSGAEVKKPGESLKISCQGSGYSFNNYYIGWVR QMPGKGLEWMVIIYPGDSDTKYSPSFQGQVTVSADRSI NTAYLQWSSLRASDTAVYYCVRMDTVTTGAGVGFNW FFDLWGRGTLVTVSS |
| VL | SEQ ID No. 8 | EIVLTQSPATLSVSPGERATLSCRASQSVTSYLAWYQQK PGQAPRLLIYGASSRATGVPARFSGSGSETEFTLTISSLQS EDFAVYYCQQYNDWPAITFGQGTRLENK |

**Table 2 CDR sequences of antibody TRN1021**

| | CDR | SEQ ID NO: | Sequence |
|---|---|---|---|
| VH | CDR1 | 1 | GYSFNNYY |
| | CDR2 | 2 | IYPGDSDT |
| | CDR3 | 3 | VRMDTVTTGAGVGFNWFFDL |
| VL | CDR1 | 4 | QSVTSY |
| | CDR2 | 5 | GAS |
| | CDR3 | 6 | QQYNDWPAIT |

| | | | |
|---|---|---|---|
| 5. Purification and analysis of expressed antibodies | | | |

After the above transfected cells were cultured for 96 h, a transfection supernatant was collected and centrifuged at 4000 rpm and 4°C for 1 h to remove cell debris. Protein A Agarose affinity chromatography was performed overnight according to the manufacturer's instructions, and the collected supernatant slowly passed through a Protein A Agarose affinity chromatographic column to allow sufficient binding of the antibody to the column. After being washed with 60 mL of PBS, the bound antibody was eluted with an elution buffer (0.1 M Gly-HCl buffer, pH=2.5), an effluent was collected, placed in Amicon Ultra-30 Centrifugal Filters (MerckMillipore) containing 1 mL of 1 M Tris-HC buffer (pH=9.0), and centrifuged at 5000 g and 4°C for 20 min to concentrate the protein, the above PBS was added to the Amicon Ultra-30 Centrifugal Filters, the mixture was centrifuged at 3500 g and 4°C for 20 min, the old buffer was replaced with a new equilibration buffer, and the above operation was repeated 3 times to obtain 1 mL of concentrated antibody protein (fully human monoclonal antibody TRN1021). Discontinuous vertical electrophoresis was performed by using the Mini-Protein cell III system (Bio-Rad). An antibody sample and a loading buffer were mixed in a ratio of 5: 1, the reducing sample was boiled for 5 min after mixing, and 10 µL of sample was placed in each well and subjected to electrophoresis for about 60 min. The sample was stained with Coomassie brilliant blue R-250 for more than 30 min, and decolorized with a decolorizing solution until the background was clean. The purification effect was observed and analyzed by SDS-PAGE protein electrophoresis.

Results are shown in Fig. 2. After purification, both the non-reducing and reducing antibody proteins TRN1021 show clear bands on the SDS-PAGE map, in which lane 1 indicates the non-reducing antibody protein, lane 2 indicates Marker, and lane 3 indicates the reducing antibody protein. The size of the non-reducing band (i.e., antibody) is about 180 kD, and the reducing sample is cleaved to a heavy chain of about 65 kD and a light chain of about 25 kD, respectively, with few other miscellaneous bands. The activity and function of the purified antibody TRN1021 is then reidentified by ELISA, etc.

### Example 2: comparison of expression levels of TRN1021 and TRN1022 antibodies in HEK293 cells

### 1. Construction of an expression vector for the recombinant antibody and expression of antibody

The procedure was the same as "4. Construction of an expression vector for the recombinant antibody and expression of antibody" of Example 1.

### 2. Purification and analysis of antibody protein

The procedure was the same as "5. Purification and analysis of expressed antibodies" of Example 1.

### 3. Calculation of average expression level of antibody

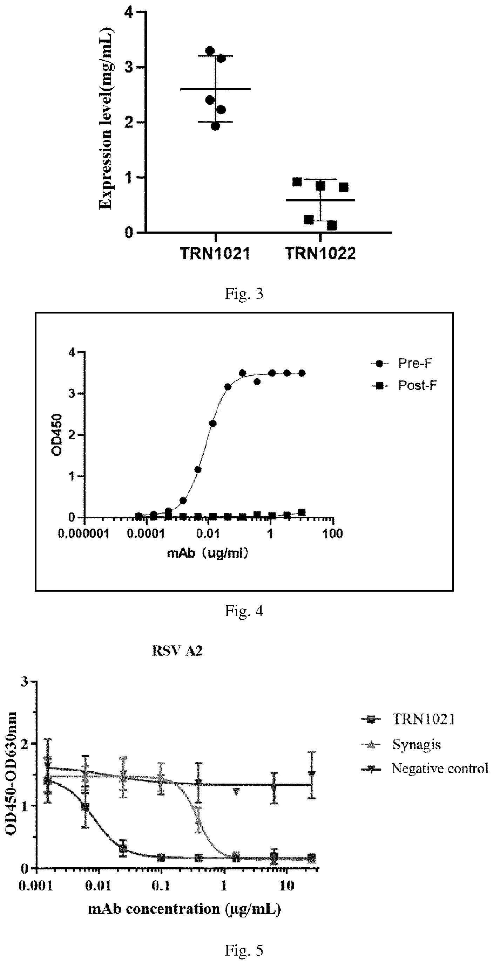

5 batches of antibodies TRN1021 and 5 batches of antibodies TRN1022 (see CN110016079A) were respectively produced. Protein concentrations of the purified antibodies were detected by using a micro-ultraviolet spectrophotometer, and average expression levels of the antibodies were calculated according to the total addition amount of medium. The antibody production information and average expression levels are shown in Table 3 and Fig. 3.

It can be seen from the data in Table 3 that the expression levels of antibody TRN1021 and antibody TRN1022 are significantly different. TRN1021 is more easily expressed, with an average expression level of 2.603 mg/L, while an average expression level of TRN1022 is only 0.588 mg/L.

**Table 3 Production information and average expression levels of antibody TRN1021 and antibody TRN1022**

| **Antibody** | **Medium volume (mL)** | **Batch** | **Purified antibody concentration (mg/mL)** | **Antibody volume (µL)** | **Total antibody (mg)** | **Average expression level (mg/L)** |
|---|---|---|---|---|---|---|
| TRN1021 | 250.000 | Batch 1 | 0.805 | 600 | 0.483 | 2.603 |
| | 4250.000 | Batch 2 | 11.174 | 1200 | 13.409 | |
| | 1750.000 | Batch 3 | 4.804 | 1200 | 5.765 | |
| | 1500.000 | Batch 4 | 5.573 | 600 | 3.344 | |
| | 1000.000 | Batch 5 | 4.011 | 600 | 2.406 | |
| TRN1022 | 750.000 | Batch 1 | 1.235 | 500 | 0.618 | 0.588 |
| | 125.000 | Batch 2 | 0.026 | 600 | 0.016 | |
| | 15625.000 | Batch 3 | 9.552 | 1500 | 14.328 | |
| | 9000.000 | Batch 4 | 6.920 | 1100 | 7.611 | |
| | 2000.000 | Batch 5 | 0.770 | 600 | 0.460 | |

### Example 3: Detection of the activity and function of anti-RSV antibody

### 1. Binding activity of antibody

The binding activity of the expressed and purified antibody TRN1021 was tested by the same ELISA method mentioned above:
RSV Pre-F and RSV Post-F proteins were respectively coated with a carbonate coating buffer on an ELISA 96-well plates at 4°C overnight. The plate was washed with PBST, the blocking solution was added, and the proteins were blocked at 37°C for 2 h or at 4°C overnight. The anti-RSV antibody sample TRN1021 (primary antibody) obtained in Example 1 was diluted with a blocking solution by 3-fold gradient starting at 1 µg/well for 12 gradients. A positive control was composed of a positive plasma sample stock solution (1: 50) and the RSV monoclonal antibody RSV3216 (B016, Abcam, #ab24011) (1: 1000), and added to the plate according to 100 µL/well. A negative control was composed of a negative plasma sample stock solution (1: 50) and an irrelevant antibody (TRN006) at a concentration of 0.5 µg/mL, and added to the plate according to 100 µL/well. 100 µL of blocking solution was added to each blank well. The samples were incubated at 37°C for 1 h. The plate was washed with PBST, 100 µL of goat anti-human IgG-HRP and goat anti-mouse IgG-HRP (secondary antibody) diluted with a blocking solution by 1: 10000 was added to each well, and the samples were incubated at 37°C for 1 h. The plate was washed with PBST, 100 µL of TMB was added to each well in the dark, the plate was placed at 37°C for 5 min, and 2 M sulfuric acid was added to terminate the reaction. OD values were measured at a dual-wavelength of 450/630 nm and calculated.

It can be seen from Fig. 4 that the expressed and purified fully human anti-RSV-F protein monoclonal antibody TRN1021 binds to the purified RSV Pre-F protein, and the above binding is dose-dependent, which indicates that the binding of the antibody TRN1021 to RSV Pre-F is specific. While the expressed and purified fully human anti-RSV-F protein monoclonal antibody TRN1021 had no specific binding activity to the purified RSV Post-F protein.

### 2. In vitro micro-neutralizing activity of antibody

A 3-fold gradient diluted solution of antibody TRN1021 was added to a HEp-2 cell medium in a 96-well microtiter plate according to a volume of 50 µL/well. Then, 50 µL of RSV A2 strain was diluted in the HEp-2 cell medium to a concentration of 2×10⁴ PFU/mL, the same operation was performed in control wells containing the HEp-2 cell medium only, and the plate was placed in an incubator with 5% CO₂ at 37°C for 2 h. 0.1 mL of prepared Hep-2 cell suspension (2×10⁵ cells/mL) was placed in cell well, and the plate was placed in the incubator with 5% CO₂ at 37°C for incubation. The cytopathic effect (CPE) was observed every day with an inverted microscope, and the determination was made when the area of cell syncytial lesions in the virus control well under the visual field accounted for more than 80% of the single well bottom area. A supernatant was removed, the plate was washed twice with PBST, and 100 µL of fixing solution containing 80% acetone was added to each well to fix the cells for 15-30 min. The fixing solution was removed, the plate was washed twice with PBST, and 300 µL of blocking solution was added to each well to block the cells at 37°C for 2 h. The plate was washed 3 times with PBST, 100 µL of RSV050-HRP (diluted with a blocking solution by 1: 5000) was added to each well, and the cells were incubated at 37°C for 1 h. The plate was washed 5 times with PBST, 100 µL of TMB was added to each well, the plate was placed at the room temperature for 5-10 min, 50 µL of 2 M H₂SO₄ was added immediately to terminate the reaction, the plate was read with a microplate reader, and OD values were measured at a dual-wavelength of 450-630 nm. If a measured OD value of diluted antibody sample is less than half of an OD value of 100CCID₅₀ virus control well, it is defined that the antibody at this concentration has neutralizing activity.

A response-variable slope curve was fitted by using log (inhibitor), and IC₅₀ values were calculated according to a non-linear fitting algorithm in Graphpad Prism. An IC₅₀ value represents the concentration of antibody TRN1021 required to reduce the absorbance measured at 450-630nm by 50%.

Results in Fig. 5 shows that equal amount of antibody TRN1021 and palivizumab (Synagis^{®}) have neutralizing activity to RSV A2 strain, the neutralizing titer of antibody TRN1021 is higher, and the minimum neutralizing concentration of antibody TRN1021 detected in the in vitro micro-neutralizing activity assay is only 0.02 µg/mL.

### 3. Affinity of antibody

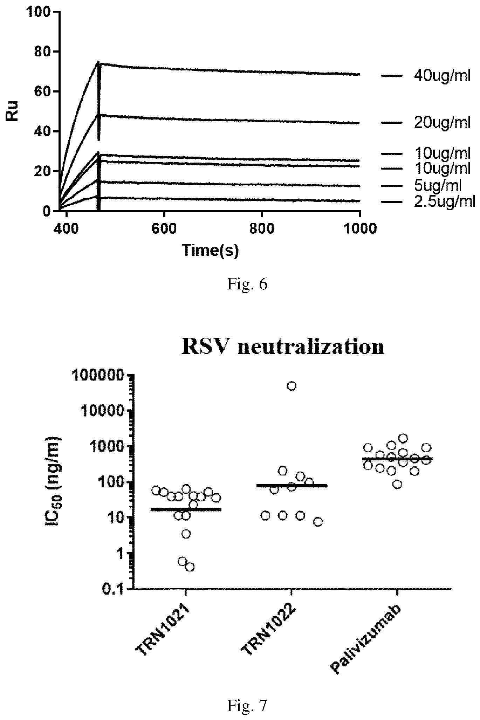

Through the surface plasmon resonance (SPR) test, anti-human IgG (Fc) was coupled to both channels of a CM5 chip by amino coupling, the anti-RSV F protein antibody was captured as a ligand, and finally channel 1 was coupled to 5485.4 RU, and channel 2 was coupled to 5622.4 RU. The concentration of captured TRN1021 was 1 µg/mL, and the binding time was 130 s. The RSV F protein at different concentrations (2.5 µg/mL, 5 µg/mL, 10 µg/mL, 20 µg/mL, and 40 µg/mL) was used as an analyte to flow through a detection channel, the binding time was 90 s, and the dissociation time was 600 s. A chip regeneration solution was 3 M MgCl₂, and the regeneration time was 30 s. Kinetic analysis (calculation of dissociation constants (KDs)) was performed using Biacore X100 Evaluation Software (2.0.1). Results are shown in Fig. 6.

The results of the surface plasmon resonance (SPR) test of two RSV F proteins (RSV Pre-F protein and RSV Post-F protein) show that the antibody TRN1021 does not bind to the post-fusion F protein (RSV Post-F) and binds to the antigen, i.e., the pre-fusion F protein (RSV Pre-F), with an equilibrium dissociation constant as low as 10⁻⁹ M. It indicates that the natural fully human anti-RSV monoclonal antibody TRN1021 can specifically bind to Pre-F.

### 4. Analysis of competition for antigen

The competition of natural fully human anti-RSV monoclonal antibodies for antigens, i.e., the RSV Pre-F protein, was analyzed by the surface plasmon resonance (SPR) method.

The antigen Pre-F protein was used to capture antibodies. First the antibody TRN1021 at a concentration of 50 µg/mL was used as an analyte to flow through a detection channel, the binding time was 180 s, and then Synagis^{®} at the same concentration was used as a second analyte to flow through the detection channel for binding and dissociation. A chip regeneration solution was glycine with a pH of 1.5, and the regeneration time was 30 s. Then, the antigen Pre-F protein was used to capture antibodies. First, Synagis^{®} at a concentration of 50 µg/mL was used as an analyte to flow through the detection channel, the binding time was 180 s, and then the antibody TRN1021 at the same concentration was used as a second analyte to flow through the detection channel for binding and dissociation.

Results show that a binding-dissociation curve of the antibody and the antigen is not affected by the second analyte when the second analyte flows through the detection channel, which indicates that TRN1021 and Synagis^{®} recognize different epitopes.

### Example 4: broad-spectrum neutralizing activity of neutralizing antibodies

The virus neutralization test was used to test the ability of the target antibody TRN1021 to inhibit the infection of HEp-2 cells by different RSV strains. In this experiment, 3 laboratory standard strains RSV A2, RSV Long, RSV 9320, and 12 RSV strains CL9325, CL8879, CL9133, CL9574, CL6477, CL8938, CL6495, CL0007, CL0014, CL0041, CL0042 and CL00J3 that were separated from clinical samples were used. The time span of clinical virus sample collection was 5 years (provided by Chongqing Children's Hospital). A total of 15 RSV strains were used for virus broad-spectrum neutralization test, which included all subtypes of RSV (two subtypes A and B), i.e., 10 RSV A strains and 5 RSV B strains. Results (see Table 4) show that the TRN1021 antibody can neutralize the laboratory standard strains RSV A2, RSV Long, RSV 9320 and 12 RSV strains CL9325, CL8879, CL9133, CL9574, CL6477, CL8938, CL6495, CL0007, CL0014, CL0041, CL0042, and CL00J3 that are separated from clinical samples, with IC₅₀ ranging from 0.0004 µg/mL to 0.0631 µg/mL, which indicates that the antibody TRN1021 has a very strong broad-spectrum neutralizing activity.

**Table 4 Broad-spectrum neutralization test of anti-RSV monoclonal antibodies (IC₅₀ µg/mL)**

| RSV sample | Subtype | IC₅₀ µg/mL |
|---|---|---|
| RSV A2 | A | 0.0229 |
| RSV Long | A | 0.0391 |
| SDF9320 | B | 0.0006 |
| CL9325 | A | 0.0114 |
| CL8879 | A | 0.0517 |
| CL9133 | A | 0.0035 |
| CL9574 | B | 0.0382 |
| CL6477 | B | 0.0004 |
| CL8938 | A | 0.0114 |
| CL6495 | A | 0.0525 |
| CL0007 | A | 0.0584 |
| CL0014 | A | 0.0631 |
| CL0041 | B | 0.0403 |
| CL0042 | B | 0.0357 |
| CL00J3 | A | 0.0392 |

The broad-spectrum neutralization capacity of the antibody TRN1021 was analyzed. The antibody TRN1022 (see CN110016079A) and the commercially available drug palivizumab (Synagis^{®}) were used as controls, and their geometric mean IC₅₀ values are shown in Fig. 7. Results show that geometric mean IC₅₀ values (represented by black horizontal lines in the figure) of the antibody TRN1021 and the antibody TRN1022 are less than that of the commercially available monoclonal antibody palivizumab, which indicates that the broad-spectrum neutralization activity of the antibody TRN1021 or the antibody TRN1022 is better that of palivizumab. While the geometric mean IC₅₀ value of the antibody TRN1021 is less than that of the antibody TRN1022, which indicates that the broad-spectrum neutralizing activity of the antibody TRN1021 is better than that of the antibody TRN1022.

### Example 5: pharmacodynamic study in animals

The efficacy of the antibody TRN1021 in animals was evaluated by using cotton rats, the standard animal model for efficacy evaluation of anti-RSV monoclonal antibodies. In the multi-dose study, experimental groups were intramuscularly injected with the antibody TRN1021 at different doses. One day after administration, the cotton rats were intranasally inoculated with RSV A2 strain or RSV 9320 strain. Four days after inoculation, virus titers in the lung and nose tissues and the concentration of antibody TRN1021 in the blood of cotton rats were evaluated.

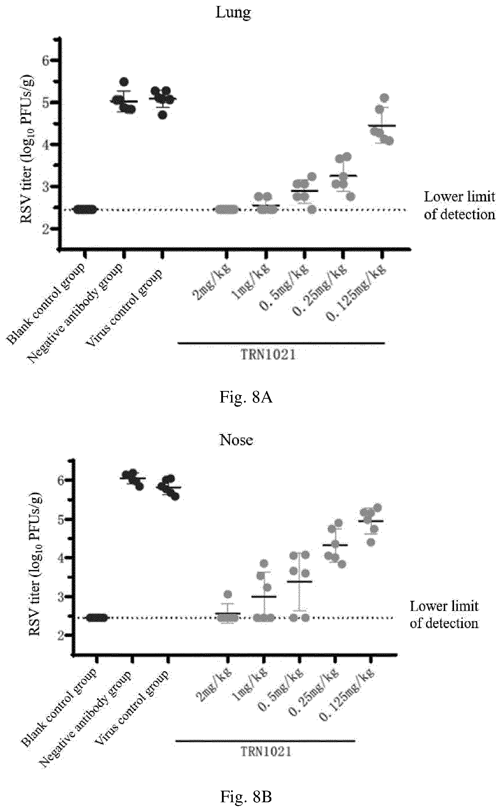

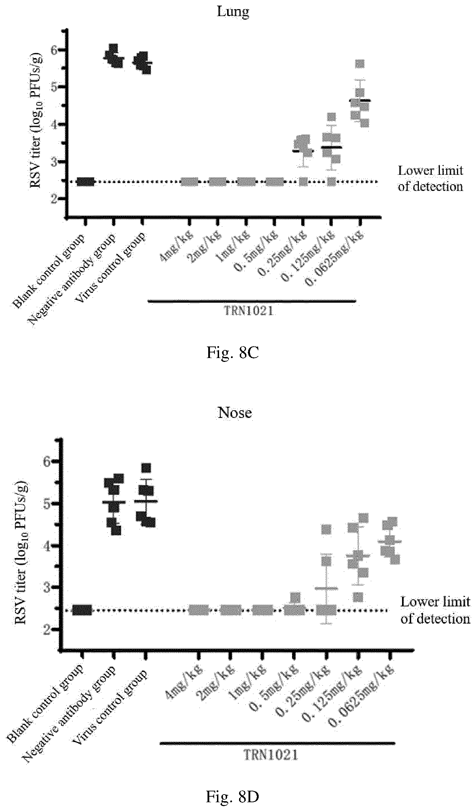

Results (see Fig. 8) show that the inhibitory effect on virus titer increases with the increase of antibody dose. When the concentration of antibody TRN1021 reaches 2 mg/kg, the antibody can 100% effectively inhibit virus infections in the lung and nasal tissues of the cotton rats inoculated with RSV A2 strain (see Fig. 8A and Fig. 8B). When the concentration of antibody TRN1021 reaches 1 mg/kg, the antibody can 100% effectively inhibit virus infections in the lung and nasal tissues of the cotton rats inoculated with RSV 9320 strain (see Fig. 8C and Fig. 8D). It can be calculated according to a non-linear fitted dose-effect curve that, the plasma concentration (i.e., EC₉₀) of the drug is 1.86 µg/mL when the inhibition rate of RSV A2 strain in the lung tissue of the cotton rat reaches 90%, and the plasma concentration (i.e., EC₉₀) of the drug is 1.83 µg/mL when the inhibition rate of RSV 9320 strain in the lung tissue of the cotton rat reaches 90%.

The dose-effect linear relationship of the above pharmacodynamic study shows that when the plasma concentration (i.e., EC₉₀) of the drug is 1.86 µg/mL, the inhibition rate of RSV in the tissue of the cotton rat reaches 90%, that is, the antibody TRN1021 has a very good protective effect on individuals infected with RSV

Although the present disclosure has been described in detail above with general description and specific embodiments, some modifications or improvements may be made on the basis of the present disclosure, which will be obvious to those skilled in the art. Therefore, these modifications or improvements made without departing from the spirit of the present disclosure shall fall within the scope of protection claimed by the present disclosure.

## Claims

1. A separated antibody or antigen-binding fragment thereof that specifically binds to the respiratory syncytial virus (RSV) surface fusion glycoprotein, wherein the antibody or antigen-binding fragment thereof comprises:
(a) the following complementarity determining regions (CDRs) of a heavy chain variable region:
CDR1, comprising a sequence shown as SEQ ID NO: 1;
CDR2, comprising a sequence shown as SEQ ID NO: 2;
CDR3, comprising a sequence shown as SEQ ID NO: 3,
(b) the following CDRs of a light chain variable region:
CDR1, comprising a sequence shown as SEQ ID NO: 4;
CDR2, comprising a sequence shown as SEQ ID NO: 5;
CDR3, comprising a sequence shown as SEQ ID NO: 6.

2. A separated antibody or antigen-binding fragment thereof that specifically binds to the RSV surface fusion glycoprotein, wherein the antibody or antigen-binding fragment thereof comprises 3 CDRs of a heavy chain variable region shown as SEQ ID NO: 7, and 3 CDRs of a light chain variable region shown as SEQ ID NO: 8;
preferably, the heavy chain variable region is selected from:
(1) a sequence comprising the heavy chain CDRs according to claim 1 and having at least 80% identity to SEQ ID NO: 7; or
(2) a sequence shown as SEQ ID NO: 7;
preferably, the light chain variable region is selected from:
(1) a sequence comprising the light chain CDRs according to claim 1 and having at least 80% identity to SEQ ID NO: 8; or
(2) a sequence shown as SEQ ID NO: 8;
more preferably, the antibody or antigen-binding fragment thereof comprises a heavy chain variable region and a light chain variable region, wherein the heavy chain variable region comprises the sequence shown as SEQ ID NO: 7, the light chain variable region comprises the sequence shown as SEQ ID NO: 8.

3. The separated antibody or antigen-binding fragment thereof according to claim 1 or 2, wherein the antibody is an IgG antibody;
preferably, the antigen-binding fragment is selected from Fab, Fab'-SH, Fv, scFv, or (Fab')2 fragments;
preferably, the antibody or antigen-binding fragment thereof comprises a constant region sequence, wherein at least a portion of the constant region sequence is a human consensus constant region sequence.

4. A nucleic acid molecule encoding the antibody or antigen-binding fragment thereof according to any one of claims 1 to 3.

5. A vector comprising the nucleic acid molecule according to claim 4;
preferably, the vector is an expression vector.

6. A host cell comprising the vector according to claim 5;
preferably, the host cell is a prokaryote or eukaryote;
preferably, the host cell is selected from an *Escherichia coli* cell, a yeast cell, a mammalian cell, and other cells applicable to the preparation of an antibody or antigen-binding fragment thereof;
preferably, the mammalian cell is a CHO cell, HEK293 cell or COS cell.

7. A production method of an antibody or antigen-binding fragment thereof that binds to the RSV surface fusion glycoprotein, comprising culturing the host cell according to claim 6 under conditions suitable for expression of a nucleic acid encoding the antibody or antigen-binding fragment thereof according to any one of claims 1 to 3, optionally separating the antibody or antigen-binding fragment thereof, and optionally collecting produced antibodies or antigen-binding fragments thereof.

8. An antibody or antigen-binding fragment thereof prepared by the method according to claim 7.

9. A pharmaceutical composition comprising the antibody or antigen-binding fragment thereof according to any one of claims 1 to 3 and 8 and a pharmaceutically acceptable carrier;
preferably, the pharmaceutical composition also comprises other therapeutic agents.

10. Use of the antibody or antigen-binding fragment thereof according to any one of claims 1 to 3 and 8 in the preparation of a drug for preventing or treating an RSV infection-associated disease or symptom;
preferably, the RSV is selected from one or more of RSV type A and RSV type B.

11. A method for preventing or treating an RSV infection-associated disease, comprising the following steps: administering an effective amount of the antibody or antigen-binding fragment thereof according to any one of claims 1 to 3 and 8 to a subject in need; preferably, the RSV is selected from one or more of RSV type A and RSV type B.

12. A method for detecting the presence of RSV in a sample, comprising a step that contacting the sample with the antibody or antigen-binding fragment thereof according to any one of claims 1 to 3 and 8.
